(19) Europäisches Patentamt

European Patent Office

Office européen des brevets

(11) Publication number: **0 624 598 A1**

# EUROPEAN PATENT APPLICATION

(21) Application number: **93401192.5**

(22) Date of filing: **10.05.93**

(51) Int. Cl.⁵: **C07K 15/04**, C12P 21/08, A61K 37/02

(43) Date of publication of application:
**17.11.94 Bulletin 94/46**

(84) Designated Contracting States:
**FR**

(71) Applicant: **INSTITUT PASTEUR**
**25-28, rue du Docteur Roux**
**F-75724 Paris Cédex 15 (FR)**

(72) Inventor: **Budkowska, Agata**
**7 avenue Beauséjour**
**F-92140 Clamart (FR)**

(74) Representative: **Desaix, Anne et al**
**Ernest Gutmann - Yves Plasseraud S.A.**
**3, rue Chauveau-Lagarde**
**F-75008 Paris (FR)**

(54) **Hepatitis B-binding factor.**

(57) The present invention relates to the isolation and characterization of a binding factor of the Hepatitis B virus from normal human sera, as well as monoclonal antibodies directed against the HBV-binding factor. More particularly, the present invention relates to a binding factor of the Hepatitis B virus which inhibits the binding of said virus to membranes on hepatocytes thus preventing Hepatitis B virus from further infecting the liver.

Rank Xerox (UK) Business Services
(3.10/3.09/3.3.4)

The present invention relates to the isolation and characterization of a binding factor of the Hepatitis B virus from normal human sera which interacts with pre-S1 and pre-S2 epitopes of the viral envelope proteins of the Hepatitis B virus, located within the domains involved in the recognition of hepatocyte receptors. More particularly, the present invention relates to a binding factor of the Hepatitis B virus which inhibits the binding of said virus to membranes on hepatocytes thus preventing Hepatitis B virus from further infecting the liver.

Description of the Prior Art

Hepatitis B is a public health problem of worldwide importance. In far East-Asia and tropical Africa chronic carriers of the hepatitis B virus (hereafter being referred to as HBV) represents a large proportion of the population and chronic active hepatitis and liver cirrhosis remain major causes of mortality. Epidemiological studies have clearly associated HBV with hepatocellular carcinoma, which is one of the most common cancers in the world. Beasly et el., Viral Hepatitis and Liver Disease, (ed. Vyas) 209-224(Grune & Stratton 1984).

The pathological consequences of HBV infection, as well as the mechanism of liver damage are not fully understood. The virus is not cytolytic and the host immune response to viral antigens present at the liver cell membrane plays a major role in the pathogenesis of HBV-related liver diseases.

During HBV infection in humans virus particles are present in large quantities in the blood of the infected individual. The presence of these complete virions is indicative of active viral multiplication in the liver. Besides the complete virion or Dane particle, 27 nm particles which characteristically carry an outer coat (HBcAg) and inner spherical core and 22 nm particles which are related to the outer surface or coat (HBsAg) of the hepatitis virus are present in sera of infected humans.

Many advances have been made over the past ten years in isolating the major viral proteins of HBcAg and HBsAg, identifying the amino acid sequences encoding the proteins and recombinantly produced the envelope proteins for vaccine purposes.

Thus, it has already been established in the art that the HBV envelope consists of three distinct coterminal proteins which are encoded by a single envelope gene. The S gene encodes for HBsAg and the pre-S region consists of both pre-S1 and pre-S2. Both pre-S proteins and their respective antibodies can be detected during HBV infection. Furthermore, several other poorly characterized parts of the pre-S gene products have been identified including receptors on the surface for polymerized human serum albumin and hepatocyte receptors. See, Harrison's Principles of Internal Medicine, 12th ed. Wilson et al. Editors McGraw Hill, N.Y. (1991).

The domains of these proteins encoded by the pre-S region of the viral genome represent potential attachment sites of HBV to the hepatocyte, since pre-S1 and pre-S2 antibodies neutralize infectivity in vitro Neurath et al., Vaccine, 7, 234-246 (1989). Furthermore, experimental models have illustrated this neutralization effect. See Itoh et al., P.N.A.S. USA, 83, 9174-9178 (1986) and Neurath et al., Vaccine, 7, 234-246 (1989). Moreover, pre-S1 and pre-S2 antibodies inhibit the binding of virions to isolated hepatocyte membranes as described by Pontisso et al., J. Virol, 63, 1981-1988 (1989) and Pontisso et al., Virology, 137, 522-530 (1989).

Neurath et al. Cell, 46 426-436 (1986) have further demonstrated that both pre-S1 and pre-S2 encoded domains of the HBV envelope proteins are involved in the recognition of hepatocyte receptors and identified the HBV binding site as being located within the amino acid sequence of 21-47 of the pre-S1 domain, as well as an auxiliary binding site within the pre-S2 domain located within amino acids 12-145.

Although viral sites for the attachment to hepatocytes have been described, the cellular receptor(s) for HBV have not yet been clearly identified and thus biochemical events leading to the infection remain unknown today.

Cells other than hepatocytes also have receptors for the pre-S1 region. These cells include hematopoietic cells of the B lymphocyte lineage, peripheral blood lymphocytes and some noon-human SV-40 transformed cell lines. See, for example, Neurath et al. Vaccine, 7, 234-246 (1986); Neurath et al. Vaccine, 7, 234-246 (1989); Neurath et al., Virology, 178, 631-634 (1990); Neurath et al., Virology, 173, 448-457 (1990); and Neurath et al., J. Exp. Med., 175, 461-469(1992). Moreover, the pre-S1 domain of the large envelope protein has a partial sequence homology with the Fc moiety of the alpha chain of IgA as described by Neurath et al., Virology, 178, 631-634 (1990), and a common receptor for the attachment of HBV to human liver cells has been proposed by Pontisso et al., J. Gen. Virol., 73 2041-2045 (1992). However, Franco et al., J. Exp. Med., 175, 1095-1105(1992) describe that the transferrin receptor might play a role in the binding of HBV to hepatocytes via a pre-S2 sequence since this domain is involved in the binding of HBsAg particles to T cells. It has also been suggested that interleukin 6 containing recognition

2

sites for the pre-S1 domain could mediate HBV-cell interaction. See for example, Neurath et al., J. Exp. Med., 175, 461-469(1992).

Vaccines can aid to prevent the infection of an individual from obtaining hepatitis B virus, however the problem that remains to be solved is how to treat a person already having hepatitis B viral infection which reaches a clinically aggressive state such as in chronic active hepatitis.

Chronic active hepatitis is a disorder of diverse etiologies which may be characterized by continuing necrosis, active inflammation and fibrosis which may lead to liver failure, cirrhosis and death. It is believed that multiple etiologic agents may initiate chronic active hepatitis among which is the hepatitis B virus. The course of chronic active hepatitis is variable and the disease may persist for long periods without clinically overt liver disease. However, continuing hepatocellular necrosis to cirrhosis is generally the rule. Death usually occurs as a result of liver failure and hepatic coma or a complication of cirrhosis.

Treatment of chronic active hepatitis is limited to the use of interferon-alpha, azathipene, D-penicllamine, cyclophosphamide, interleukin 2, acyclovir, adenine arabinoside and adenine arabinoside monophosphate. However, in the majority of patients with chronic active hepatitis, none of these agents have been shown to be effective. See, Harrison's Principles of Internal Medicine, 12th ed. Wilson et al. Editors, McGraw Hill, N.Y. (1991).

Persistence of HBsAg in the sera of patients infected with chronic active hepatitis is found in between 20 to 30 percent of patients suggesting that persistent hepatitis B virus infection may be related to this disease.

The discovery of HBV-binding factor involved in the recognition of hepatocyte receptors may aid in the treatment of cases of chronic active hepatitis and liver deterioration by inhibiting the attachment of Hepatitis B virus to human hepatocyte membranes. Additionally, the HBV-binding factor of the present invention can be used to prevent hepatocellular carcinoma and liver cirrhosis as a result of HBV infection.

## SUMMARY AND OBJECTS OF THE PRESENT INVENTION

Thus, it is an object of the present invention to provide an isolated HBV-binding factor which interacts with the preS-1 and preS-2 epitopes of the viral envelope proteins located within the domains involved in recognition of hepatocyte receptor(s).

It is another object of the present invention to provide an HBV-binding factor which inhibits the binding of the Hepatitis B virus to the membranes of human hepatocytes, thus preventing this virus from further infecting the liver.

It is another object of the present invention to provide an HBV-binding factor that is useful for the treatment of chronic acute hepatitis and other liver diseases associated with chronic acute hepatitis, as well as the prevention of hepatocellular carcinoma and liver cirrhosis.

It is a further object of the present invention to provide a procedure for isolating said HBV-binding factor that is a hepatocyte receptor(s) which mediates the attachment of HBV to liver cells.

It is yet a further object of the present invention to provide monoclonal antibodies directed to the HBV-binding factor of the present invention.

In one of its compositional aspects, the present invention is directed to a novel and useful HBV-binding factor, which binding factor can be characterized as having a molecular weight of 50 kDA, is a glycoprotein derived from human sera, has an isoelectric point of 7.13 and is immunologically related to a membrane component of human hepatocytes, but not related immunologically to hepatocytes of species that are not susceptible to hepatitis B virus infection.

In another compositional aspect, the present invention is directed to a novel and useful HBV-binding factor, which additionally contains N-acetylgalactosamine and N-acetylglucosamine residues, requires intact disulfide bonds, hydrophobic bonds and additional non-covalent linkages to maintain the conformation necessary for HBV recognition, represents a soluble form or fragment of the cellular HBV receptor, being antigenically related to HBV-binding factor in the liver cell membrane, is stable at 37°C and is not a protease degrading pre-S determinants.

In one of its procedural aspects, the present invention relates to a process for isolating an HBV-binding factor, said process comprising the steps of:

(a) precipitating human sera with ammonium sulfate to produce globulins;

(b) dialyzing said precipitated globulins to remove the ammonium sulfate;

(c) placing said dialyzed globulins over an anion-exchange column, particularly DEAE-cellulose;

(d) eluting fractions containing HBV-binding factor from said anion-exchange column;

(e) incubating said eluted fractions containing HBV-binding factor at 4°C for at least two weeks;

(f) placing said incubated fractions over a gel filtration column such as Sephacryl S-200 column;

(g) eluting said fractions containing HBV-binding factor from said gel filtration column;

(h) adsorbing said eluted fractions from said gel filtration column onto beads preadsorbed with anti-Fc, followed by beads preadsorbed with anti-Fab; and

(i) isolating said HBV-binding factor from the beads.

BRIEF DESCRIPTION OF THE DRAWINGS

Fig. 1 represents an inhibition ELISA assay using pre-S1 specific Ma18/7 monoclonal antibody(□), Monoclonal antibodies 144 and 112 (○) and pre-S2 specific F124 monoclonal antibody (●) to HBV or to recombinant m-HBsAg particles by isolated HBV-binding factor of human sera.

Fig. 2 represents the reactivity of anti-pre-S1 monoclonal antibody 5a-19 on a gel with HBV preincubated with the HBV-binding factor of the present invention (lane 1) and with a control HBV preparation (lane 2).

Fig. 3A represents the HBV-binding factor of the present invention and HBV-binding factor activity corresponding to the fractions expressed in percent inhibition of anti-pre-S2 monoclonal antibody F124 corresponding to the fractions obtained from HPLC.

Fig. 3B represents the HBV-binding factor of the present invention with percent inhibition of anti-pre-S2 monoclonal antibody F124 via titration corresponding to the fractions obtained from isoelectric focusing.

Fig. 4 represents a characterization profile of the HBV-binding factor of the present invention after various treatments with enzymes and physicochemical agents; results of the treatment are presented as percent of the pre-S binding activity (inhibition of F124 monoclonal antibody) in comparison to a control untreated sample.

Fig. 5 represents an inhibition of binding curve using a variety of lectins such as Wheat Germ Agglutinin (□), Peanut Lectin (■), Concavalin A (▲), Helix Pomatia Lectin (○) and control (▫) and the HBV-binding factor of the present invention.

Fig. 6 represents an elution profile of fractions after HPLC using monoclonal antibody 9H11B3, as well as the inhibition of F124 monoclonal antibody(■) and by dot blot (+) for detection of the HBV-binding factor of the present invention.

Fig. 7A represents immunohistochemical staining of human liver hepatocytes membranes using the monoclonal antibody of the present invention.

Fig. 7B represents immunohistochemical staining of woodchuck hepatocytes membranes using the monoclonal antibody of the present invention.

Fig. 7C represents immunohistochemical staining of HepG2 cells using the monoclonal antibody of the present invention.

DESCRIPTION OF THE PREFERRED EMBODIMENTS OF THE PRESENT INVENTION

As used herein the term "HBV" means hepatitis B virus.

As used herein, the term "membrane receptor(s) or "hepatocyte receptor(s) refers to all or part of at least one receptor on the membrane of hepatocytes.

More specifically, the present invention relates to the identification and characterization of a binding factor that interacts with epitopes localized within the pre-S1 and pre-S2 domains of the viral envelope of the hepatitis B virus that appears to be a membrane receptor(s) mediating the attachment of the hepatitis B virus to the liver cell. The HBV-binding factor of the present invention inhibits the binding of HBV to hepatocyte membranes, thus preventing further virus infection.

This binding factor is identified as a glycoprotein having a molecular weight of about 50 kDa, having an isoelectric point of about 7.13 and biological activity depending on its native molecular conformation and on intact sulfhydryl bonds. This protein also inhibits the binding of pre-S1 and pre-S2 specific monoclonal antibodies to corresponding epitopes is completely inactivated at 60°C, upon treatment with sodium dodecyl sulfate, 2-mercaptoethanol or at an acidic pH.

Furthermore, the present invention relates to a process to isolate the HBV-binding factor and the production of monoclonal antibodies directed against this HBV-binding.

Prior to the discovery of the disclosed HBV-binding factor the detection of pre-S1 antibodies recognizing the amino acid sequence 29-36 of the pre-S1 region and pre-S2 antibodies (which recognizes an epitope localized in the N-terminal part of the pre-S2 domain, close to the pre-S2 glycosylation site), in sera from patients had been confirmed by use of control experiments performed on normal and pathological sera which proved the specificity of these antibodies. See, Budkowska et al. Hepatology, 12, 1271-1277(1990). However, in many of these assays it was noticed that in some normal human sera, a factor different from circulating antibodies was blocking the binding of the specific monoclonal antibodies in the pre-S1 and pre-S2 domains. This blocking effect could not be explained by the presence of circulating antibodies, nor was it due to the specificity of these assays. The masking of pre-S1 and pre-S2 region encoded epitopes has also been observed by Heerman et al. in "Interaction between native human serum and the pre-S2 domain of hepatitis B surface antigen." p. 697-700 in A.J. Zuckerman (ed.) Viral hepatitis and Liver Disease Alan R. Liss Inc, New York (1988) and Mimms et al., Virology, 176, 604-619 (1990). In these studies, the blocking activity in human sera was not associated with the presence of specific antibodies, since such activity was not removed by protein A. It was concluded that this activity was due to monomeric albumin since the the activity eluted in the same fractions by 0.5 M Bio-gel chromatography. See, Heerman et al. in "Interaction between native human serum and the pre-S2 domain of hepatitis B surface antigen." p. 697-700 in A.J. Zuckerman (ed.) Viral hepatitis and Liver Disease Alan R. Liss Inc, New York (1988).

However, upon further investigation by the present inventors it was discovered that this blocking activity was due to an HBV-binding or inhibiting factor. This HBV-binding factor was then isolated by a series of steps set forth below and then characterized with a series of experiments.

The isolation of the HBV-binding factor commences with the precipitation of globulins from normal human sera. Any human sera can be utilized provided that this sera is from a healthy individual. This sera is obtained by drawing a blood sample from a normal individual and spinning the blood at about 3,000 r.p.m.'s (1,600 x g) for about 10 minutes to separate the sera from the red blood cells.

Generally about 20 to 60 mls of sera is utilized in the precipitation step. However it is preferable to use about 50 mls of sera.

The human sera is then precipitated using between about 45-50% saturated ammonium sulfate. Usually this precipitation is performed over a period of time and requires gradual addition of ammonium sulfate to the sera under constant stirring. This precipitation also takes place at between about 4-10°C, preferably 4°C.

After precipitation, the globulins are then dialyzed against 10 mM phosphate buffer pH 7.4 to remove the ammonium sulfate. This dialysis occurs at 4°C and usually for between 24-52 hours. It is preferable to dialyze the globulins for at least 24 hours, more preferably 48 hours to ensure complete removal of the ammonium sulfate.

The dialyzed globulins are then applied to an anion-exchange column. Any anion-exhange can be used to purify the HBV-binding factor of the present invention such as DEAE-Sephacel, TEAE Cellulose, PEI Cellulose and the like. It is preferable to use a DEAE-cellulose column. Prior to sample application the anion-exchange column is preferably preequilibrated with 10 mM phosphate buffer pH 7.4. Generally, it is best to preequilibrate the column for at least one hour.

Since HBV-binding factor was found to be stable at 37°C, the anion-exchange column can be run at varying temperatures between 4°C to 30°C without protein degradation. It is preferable to perform the anion-exchange chromatography at ambient or room temperature.

About 20 to 30 ml of dialyzed globulins are then applied to the preequilibrated anion-exchange column. It is preferable to apply about 25 ml to a DEAE-cellulose column.

After application of the sample, the unbound fraction containing the HBV-binding factor is then recovered using either 10 mM phosphate buffer, pH 7.4 or 30 mM phosphate buffer, pH 7.4. It is preferable to recover the HBV-binding factor in 10 mM phosphate buffer, pH 7.4 to obtain a better purification of the HBV-binding factor at this step of purification. However, it is also possible to use 30 mM phosphate buffer, pH 7.4 to obtain a greater quantity of the HBV-binding factor with some impurities.

After the fractions are eluted, they are kept for at least 2 weeks, preferably 2-3 weeks at 4°C in order to unmask the binding factor activity. An alternative method to unmask the HBV-binding factor of the present invention is to freeze and then thaw the starting human sera about 2-5 times, preferably 3 times prior to ammonium sulfate precipitation. If this alternative freeze-thawing method is used, then the at least 2 week incubation period can be ignored, since the binding factor activity is already unmasked. Additionally, the HBV-binding factor of the present invention can be unmasked by leaving the human sera at room temperature for at least two days.

The fractions containing HBV-binding factor, as measured by ELISA using pre-S1 monoclonal antibody MA18/7 and pre-S2 monoclonal antibody F124, are then pooled and concentrated. Any concentration method known in the art via amicon, collodion bags and the like may be utilized. It is preferable to dialyze the pooled fractions against pure sucrose using a 10,000 molecular weight dialysis tubing to concentrate the fractions. The duration of dialysis is at least 4 hours or until about 5 to 10 ml of liquid containing the HBV-binding factor remains in the dialysis tubing.

The concentrated fraction containing the HBV-binding factor is then placed over a gel filtration column to remove molecules of high molecular weights. Generally about 5 to 10 ml of concentrate is utilized in this purification step. It is preferable to use a series of at least three connected Sephacryl S-200 columns (1.8 x 90 cm) for purification purposes.

Phosphate buffered saline (PBS) containing 0.1% w/v of sodium azide is used as the eluting buffer. After elution the various fractions are then analyzed for HBV-binding factor activity by inhibition ELISA using the monoclonal antibodies described above. The positive fractions are then pooled. About 30 ml of sample in buffer containing the HBV-binding factor is obtained containing approximately 50 μg/ml of the isolated HBV-binding factor protein.

The final preparations containing HBV-binding factor activity are then absorbed with insolubilized anti-IgG (anti-Fc and anti-Fab) to remove contaminating IgG and its fragments which co-purify with the HBV binding factor. Anti-Fc and anti-Fab preadsorbed on beads are used in this step. The eluent obtained after gel filtration is then mixed with anti-Fc in a conical tube and incubated at 37°C for 1 hour. The beads are then separated from the supernatant fraction by any method known in the art. It is preferable to use centrifugation for this separation step. The centrifugation speed is not critical, as long as the beads are separated from the supernatant. Usually centrifuging at about 10,000 r.p.m. for a few seconds is sufficient. The supernatant is then carefully separated from the beads. The same procedure is then performed with the anti-Fab beads using the supernatant from the anti-Fc step.

Once again, the final fractions are subjected to HBV-binding factor activity via solid phase inhibition ELISA using the monoclonal antibodies discussed above to ensure that HBV-binding activity is present.

After obtaining the purified HBV-binding factor, the protein can then be sequenced using the method described by Maxam and Gilbert, P.N.A.S., 74, 560 (1977). The purified HBV-binding factor can also be cloned into recombinant expression vectors such as the plasmid vectors of pBR322, pUc18, pUC19 and the like, bacteriophage vectors such as lambda gt10, lambda gt11, lambda gt18 and the like and/or cosmid vectors using a proper promoter and introduced into a host microorganism such as bacteria, yeast, and/or eukaryotic cells for recombinant reproduction of the HBV-binding factor protein. These methods are well known in the art and are thoroughly described by Sambrook et al in Molecular Cloning, A Laboratory Manual, Cold Spring Harbor Laboratory Press (1989).

Monoclonal antibodies can also be made directed against the HBV-binding factor. Purified HBV-binding factor from human sera can be used to immunize Balb/c mice. The purification procedure is set forth above or an alternate procedure involving precipitation with ammonium sulfate, DEAE-cellulose chromatography followed by either HPLC or preparative isoelectric focusing can also be utilized to obtain the isolated HBV-binding factor protein for immunization purposes. When HPLC is utilized in the purification step the HBV-binding factor is isolated at 50 kDA. When preparative isoelectric focusing is used, the HBV-binding factor is isolated at pH 7.13.

Mice are then immunized subcutaneously with between 0.3 to 0.7 ml of purified HBV-binding factor, preferably 0.5 ml, emulsified in complete Freunds adjuvant.

Any injection protocol known in the art can be utilized to immunize mice. It is preferable to first inject the purified HBV-binding factor emulsified in complete Freund's adjuvant, subcutaneously, followed by two intramuscular injections with incomplete Freund's adjuvant at 10-day intervals. One final injection of about 0.2 ml of purified HBV-binding factor in PBS is preferably given 3 days before fusion with NSO myeloma cells.

The monoclonal antibodies are produced according to the procedure of Kohler and Milstein, Nature, 256, 495 (1975). Hybridomas are then screened for the presence of antibodies against HBV-binding factor by ELISA on purified antigen coated plates using horse radish peroxidase (HRPO) conjugated anti-mouse immunoglobulins (Heavy + Light chains (H + L)) (Dako).

The positive hybridomas identified by the above screening method can be further subcloned by methods known in the art.

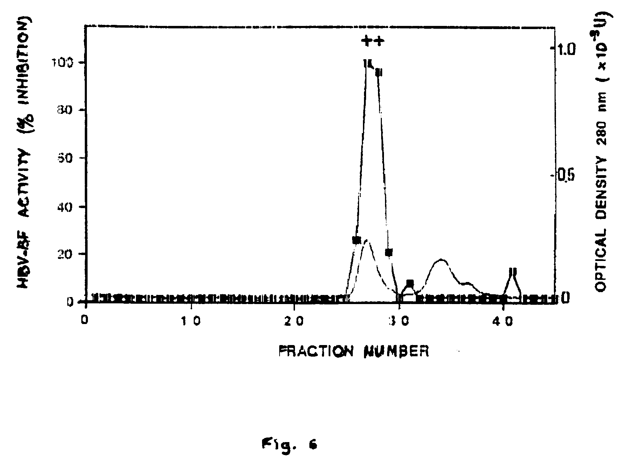

To determine whether the obtained monoclonal antibodies were reactive specifically with HBV-binding factor, the purified preparation of HBV-binding factor was preabsorbed with these antibodies prior to the reaction with pre-S containing HBsAg particles. This treatment completely abolished the pre-S binding activity of HBV-binding factor. Furthermore, the reactivity of monoclonal antibodies with HBV-binding factor was confirmed by dot-blot analysis of the HPLC fractions. Monoclonal antibody 9H11B3, representative for the group of antibodies tested, recognized the fractions with HBV-binding factor activity eluted from HPLC column as shown in Figure 6.

Monoclonal antibody 9H11B3 was deposited at the C.N.C.M. (Collection Nationale de Cultures de Microorganismes) under accession No. I1304 on May 7, 1993.

Ascites can also be produced by intraperitoneal injections of hybridoma cells to mice pretreated with 0.5 ml of pristane.

The monoclonal antibodies obtained can be further purified by absorption to and elution from their cognate antigen. The antigen (HBV-binding factor) may be coupled to a matrix such as cyanogen-bromide activated sepharose. The antibodies are further passed through the column and are retained by the corresponding antigen. The bound antibody may be further released by agents which disrupt the antigen-antibody complex such as potassium thiocyanate, low pH-buffers and the like.

Additionally, the monoclonal antibodies of the present invention may be coupled to an insoluble matrix such as a resin for use in affinity chromatography and the dialyzate obtained after ammonium sulfate precipitation may be placed over the column forming a complex with the monoclonal antibody and after removal of the non-fixed components, the HBV-binding factor is then recovered from said complex.

The HBV-binding factor isolated using the procedure set forth above, was then characterized by a variety of methods. These methods included proteolytic degradation studies, determination of molecular weight by HPLC, isoelectric focusing, digestion with subtilisin, absorption to various lectins, inactivation studies, as well as immunohistochemical staining.

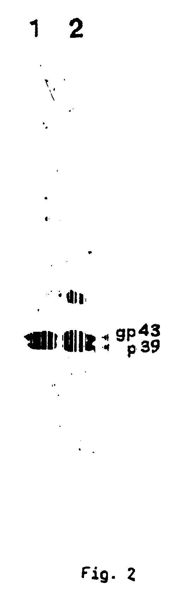

To determine if the HBV-binding factor was not a protease degrading pre-S determinants but does indeed inhibit the binding of pre-S epitopes, inhibition assays can be carried out in the presence of proteolytic enzymes such as phenylmethanesulphonyl fluoride, aprotinin and the like. The proteolytic enzyme inhibitors failed to impair the blocking activity of HBV-binding factor to pre-S epitopes. Furthermore the presence of the large envelope protein containing both pre-S1 and pre-S2 region encoded epitopes with unchanged molecular weight was evidenced by Western blot in HBV-binding factor treated viral particles, as evidenced in Figure 2.

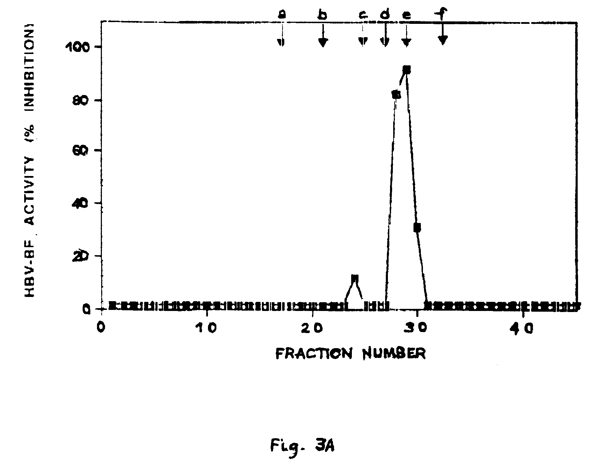

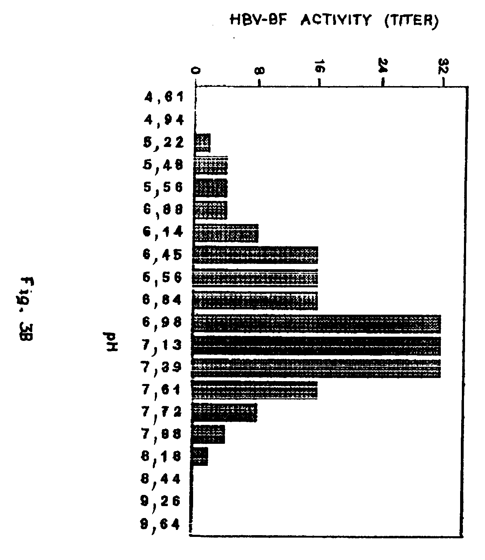

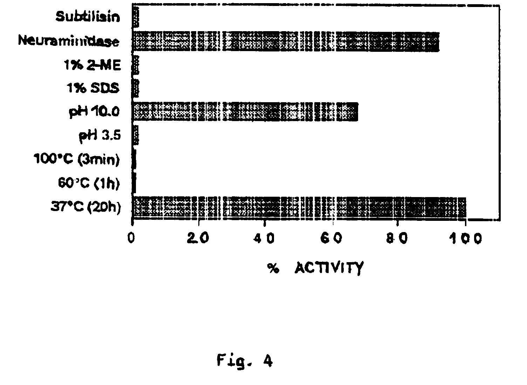

Analysis of the apparent molecular weight and isoelectric point of the HBV-binding factor revealed a molecule of about 50 kDA by size exclusion HPLC and an isoelectric point of 7.13 by isoelectric focusing. Titration of the HBV-binding factor activity by inhibition of monoclonal antibody F124 for the fractions obtained from HPLC and isoelectric focusing is illustrated in Figures 3A and 3B. Complete inactivation of HBV-binding factor was observed with either SDS or 2-mercaptoethanol as shown in Figure 4 demonstrating the requirement of intact disulfide bonds and the presence of hydrophobic bonds in HBV-binding factor which are necessary for binding to the pre-S domains. Inactivation of HBV-binding factor also occurred at high temperature and at acidic pH indicating that additional non-covalent links are essential for maintaining the conformation necessary for recognition of HBV. Other physicochemical characteristics evidenced that HBV-binding factor is a glycoprotein containing N-acetylgalactosamine and N-acetylglucosamine residues, as evidenced by the absorption of HBV-binding factor to particular lectins. See Figure 6.

Moreover, HBV-binding factor interacts with both the pre-S1 and pre-S2 domains of the viral envelope representing putative HBV attachment proteins.

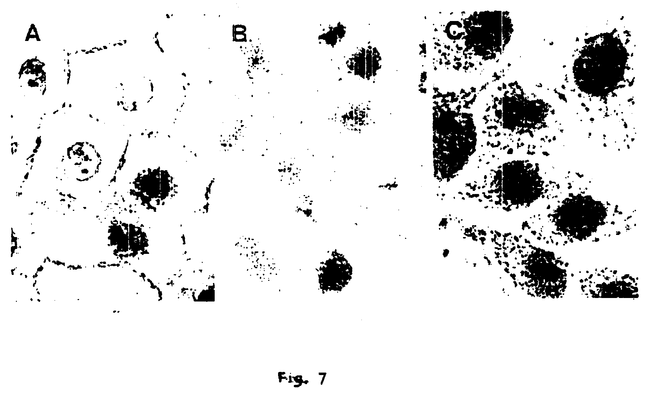

Immunocytochemical studies revealed that the monoclonal antibodies directed to the HBV-binding factor of the present invention recognized plasma membrane components of normal human hepatocytes, but did not recognize hepatocytes from other species. Monoclonal antibodies recognizing HBV-binding factor activity resulted in a thin staining pattern all along the hepatocyte membrane giving to parenchyma a honey comb pattern on human liver sections obtained from normal human liver as shown in Figure 7A. Furthermore, no staining was observed on liver sections when PBS or irrelevant antibodies were used as a control. Furthermore no staining was observed in livers of other species such as rabbit, rat, chicken, Peking duck, mouse and woodchuck as indicated in Figure 7B. HepG2 cells fixed and stained under the same conditions remained unlabeled as illustrated in Figure 7C. Therefore, the HBV-binding factor is related to a membrane component of normal human hepatocytes and is nondetectable in hepatocyte membranes of species or cells lines such as HepG2 cells non-susceptible to HBV infection.

The binding properties and the chemical composition of HBV-binding factor as well as membranous localization of the HBV-binding factor antigen illustrates that serum HBV-binding factor may represent a soluble form of cellular HBV-hepatocyte receptor(s).

The HBV-binding factor of the present invention used as a cellular HBV-hepatocyte receptor(s) is of significance for the treatment of chronic active hepatitis. For example, it is known that circulating HBV binds to the hepatocytes of the liver thereby expressing viral antigens on the liver infected cells, which expressed antigens serve as targets for human immune responses. Use of the present invention to block the binding of HBV in hepatocytes would result in a method for preventing infection and expression of this antigen in, for example, patients having chronic active hepatitis. Moreover, the HBV-binding factor of the present invention can be used to prevent hepatocellular carcinoma and cirrhosis of the liver as a result of chronic HBV infection. Therefore, use of the HBV-binding factor of the present invention for the treatment and/or prevention of HBV-related diseases such as chronic active hepatitis, hepatocellular carcinoma and liver cirrhosis is also encompassed by the present invention.

For the treatment and/or prevention of HBV-related liver diseases the HBV-binding factor is administered in a pharmaceutically acceptable amount to a person in need of such treatment. Any pharamaceutically acceptable vehicle that retains HBV-binding factor activity can be used in conjunction with the HBV-binding factor of the present invention. In this regard, it is well within the skills of a person skilled in the art to formulate such a pharmaceutical composition.

In order to further illustrate the present invention and advantages thereof, the following specific examples are given, it being understood that the same are intended only as illustrative and in nowise limitative.

EXAMPLE 1

Detection of the preS binding Protein

A. Antibodies used

Solid phase inhibition ELISA was used for the detection of antibodies directed against the pre-S1 and pre-S2 region encoded peptides in human sera according to the method described by Budkowska et al., Hepatology, 12, 1271-1277 (1990). Recombinant HBsAg particles with pre-S2 epitopes were prepared as described by Michel et al., P.N.A.S., USA 81, 7708-7712 (1984). Ma18/7 monoclonal antibody was obtained according to the method described by Heermann et al., J. Virol., 52, 396-402 (1984). Monoclonal antibody F 124 directed to the pre-S2 specific domain was obtained by the method described by Budkowska et al in J. Med. Virol., 20, 111-125 (1986). Monoclonal antibodies 144 and 112 were obtained in a commercial kit from Pasteur Diagnostics-"MonoLiza". Monoclonal antibody 39-10 was obtained according to the method described by Tillot at el., Develop. Bio. Std., vol. 57, 299-304 (1984).

Serum derived from viral particles rich in preS1 were captured on the wells of microtiter plates from HBV concentrates by means of an anti-HBs monoclonal antibody 39-10.

B. Solid Phase Inhibition ELISA

100 $\mu$l of the appropriate antigen (HBV derived from viral particles rich in pre-S1 or recombinantly produced m-HBsAg) having a concentration of 1 mg/ml was coated on a microtiter plate. The microtiter plate was then saturated with 200 $\mu$l of 1% bovine serum albumin (BSA) diluted in phosphate buffered saline (PBS). The microtiter plate was then washed with 0.05% Tween (polyoxyethylenesorbitan monolaurate) in PBS. 100 $\mu$l of sample was then added and the plates were incubated for 2 hours at 37°C. The plates were then washed with 0.05% Tween in PBS and 100 $\mu$l of antibody labeled with horse radish peroxidase was then added, followed by 100 $\mu$l of O-phenylenediamine (OPD) as a substrate. The plates were further incubated for 5 to 15 minutes and the reaction was stopped using 50 $\mu$l of 2 M sulfuric acid. Absorbance was then read at 492 nm.

The results of the inhibition assays were expressed as percent inhibition calculated from the absorbance at 492 nm and the following formula was used to calculate inhibition:

$$\% \text{ inhibition} = \frac{\text{A negative control} - \text{A test sample}}{\text{A negative control} - \text{A positive control}} \times 100$$

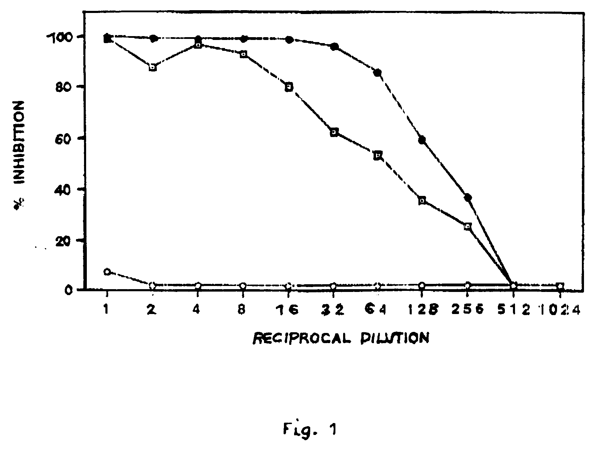

The samples were considered positive when more than 30% inhibition was obtained. The results are set forth in Figure 1.

EXAMPLE 2

Purification of The pre-S Binding Protein from Human Sera

Human blood was drawn from a healthy individual and the blood was centrifuged at 3,000 r.p.m. (1,600 x g) for 10 minutes which separated the sera from the red blood cells.

To 50 ml of human sera at 4°C diluted in an equal volume of PBS (50 ml), 100 ml of saturated ammonium sulfate (50%-final concentration) was added and the sample was stirred until a precipitate was formed. The precipitate was then diluted in PBS and dialyzed for 48 hours at 4°C against 10 mM phosphate buffer pH 7.4. 25 Ml of the dialyzed sample was then applied to a column of DEAE-cellulose (DE-52 Whatman) which was preequilibrated prior to sample application with 10 mM phosphate buffer pH 7.4. The unbound fraction containing the HBV-binding factor was recovered from the column using 10 mM of phosphate buffer pH 7.4. The collected fraction were then left for 2 weeks at 4°C to expose the HBV-binding factor activity.

An alternative method to expose HBV-binding factor activity was also performed. This method included freeze-thawing the sera obtained from the healthy human individual prior to precipitating with ammonium sulfate. The sera was first frozen and then permitted to thaw at room temperature. This freeze-thawing procedure was repeated three times.

The fractions were then measured for percent inhibition as set forth in Example 1 and fractions that were positive for HBV-binding factor activity were then concentrated by dialysis against concentrated sucrose to about 5 to 10 mls.

10 ml of dialyzed concentrate was then placed over a series of three connected Sephacryl S-200 columns (1.8 x 90 cm) and the samples were eluted in PBS containing 0.1% w/v sodium azide.

The final eluent containing the HBV-binding factor was added to beads preadsorbed with anti-FC and incubated at 37°C for 1 hour. The beads and eluent were then spun at 10,000 r.p.m. for 5 seconds and the supernatant was decanted. The supernatant was then mixed with beads preadsorbed with anti-Fab and the same procedure for anti-Fc was followed. The purified HBV-binding factor was first tested for HBV-binding factor activity and then stored at 4°C for further study.

EXAMPLE 3

Characterization of the HBV-binding Factor

HPLC

The apparent molecular mass of the HBV-binding factor was estimated by gel permeatation using the purified HBV-binding factor obtained from Example 2. HPLC was performed with a Superose 12 column (Pharmacia) at a flow rate of 12 ml/h. Fractions were collected every 2.5 min. Absorbance was detected with an HPLC spectrophotometer (Gilson, Villiers le Bel, France) connected to a CR3A integrator (Shimiadzu, Kypto, Japan). Molecular mass of the HBV-binding factor was established by comparison using standards of chymotrypsin (25 kDA), Fab (50 kDA), F(ab')$_2$ (100 kDA), IgG (150 kDa), sIgAm (410 kDa) and IgM (900 kDA).

ISOELECTRIC FOCUSING

Preparative isoelectric focusing was carried out in a liquid phase using a Rotor cell (Bio-Rad, Richmond, California). The purified HBV-binding factor obtained in Example 2 was dialyzed against 1% w/v glycine containing 1% v/v glycerol. The fractions were collected in separate tubes, according to the direction of the manufacturer (Bio-Rad), dialyzed against PBS after measurement of the pH gradient via a pH meter and analyzed for HBV-binding factor activity.

ABSORPTION ACTIVITY WITH LECTINS

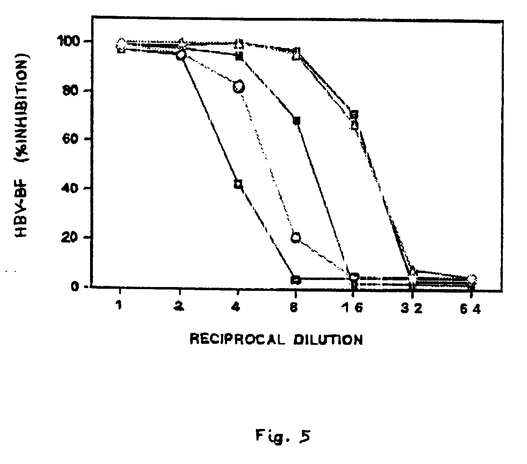

Concavalin A, Wheat germ Agglutinin and Helix Pomatia lectin were bound separately to Sepharose 6MB columns from Pharmacia(Uppsula, Sweden). Peanut agglutinin was bound to Ultrogel beads (IBF Villeneuve-la-Garenne, France). Absorption procedures were carried out according to the instructions of the manufacturer(Pharmacia or IBF). In the case of Concavalin A, 1 mM of manganese and calcium ions were added to the buffer. The results are set forth in Figure 5.

ISOLATION OF HBV AND HBsAG PARTICLES

Natural HBV particles were prepared from the plasma of a virus carrier having any serotype by pelletting the virus particles for 6 hours at 220,000 x g. The pellet was then washed three (3) times in PBS and subjected to isopycnic centrifugation in a 10-60% sucrose gradient for 26 hours at 280,000 x g. Fractions that were rich in HBV DNA banded at 38-43.5% sucrose and were analyzed for HBV using an Abbott Hepatitis B DNA kit. The desired fractions were then pooled and concentrated. The preparation was further analyzed via electron microscopy to ensure that the preparation contained mainly complete virions (Dane particles), tubules and only minute amounts of 22 nm forms. Recombinant m-HBsAg particles which contained the middle and major HBV proteins were obtained from Pasteur Vaccin and purified by the producer according to the method of Michel et al., P.N.A.S., 81, 7708-7712(1984).

DEGRADATION EXPERIMENTS

1 $\mu$g/ml of recombinant HBsAg-pre-S2 particles which were coated on plates were incubated with purified HBV-binding factor in PBS containing 0.002 M phenylmethanesulphonyl fluoride (PMSF) and/or 2000 K.I.U./ml of Aprotinin (Trasylol). The activity of HBV-binding factor was measured by the inhibition assays (see, Example 1) with and without inhibitors.

HBV particles, which were purified from serum (see above) were incubated with HBV-binding factor until pre-S epitopes were no longer detected using pre-S specific monoclonal antibodies in an Elisa according to Experiment 1. The treated and a control sample were then denatured, subjected to SDS-PAGE and then immunoblotted.

WESTERN BLOT

Control, viral serum, viral serum particles and viral particles preincubated with HBV-binding factor were solubilized in a sample buffer containing 2% sodium dodecyl sulfate (SDS), 2-mercaptoethanol (2-ME), 0.001% bromophenol blue and 20% glycerol for 2 minutes at 100°C. The proteins were then separated on 5-15% gels and electroblotted to nitrocellulose. The membrane strips were postcoated (saturated) overnight at 4°C with 5% skim milk, washed with 0.05% Tween in PBS and reacted for 1 hour at room temperature with anti-pre-S1 5a-19 monoclonal antibody diluted in 1% skim milk. This monoclonal antibody recognized the amino acid sequence pre-S1 (35-53) of the ay antigenic determinant and can be produced by the procedure set forth below. HRPO-labeled anti-mouse Ig(H+L)(Dako) served as a second antibody. After final rinsing, the blots were visualized using an enhanced chemiluminescent detection system (Amersham, Little Chalfont, UK).

MONOCLONAL ANTIBODY 5a-19

Complete virions (Dane particles) were purified from serum of HBV carrier rich in DNA from both pre-S1 and pre-S2 antigens. The serum was obtained and pelleted at 4 hours at 200,000 x g. The pellet was then suspended in PBS, filtered and subjected to an isopycnic centrifugation in a 20-60% sucrose gradient for 24 hours at 220,000 x g. Fractions that were positive for pre-S1 and viral DNA were pooled and subjected to a gradient run in 20-60% sucrose for 5 hours at 220,000 x g. Fractions that were positive for pre-S1 and HBV DNA were pooled and used for immunization.

Balb/c mice were immunized intramuscularly with 0.1 ml of viral particles emulsified in complete Freund's Adjuvant. On day 14 the mice were screened for the presence of antibodies directed to envelope proteins of HBV. On day 24 the mice were boosted intraperitoneally with 0.1 ml of viral preparation without adjuvant. Three days later the spleen cells of the mice were fused with myeloma SP2/0-Ag cells as described by Buttin et al., Current Topics in Microbiol. and Immunol., 81, 27-36 (1978). Hybrides were screened for anti-pre-S activity by solid phase ELISA with recombinant HBsAg particles rich in pre-S epitopes or synthetic pre-S peptides as described above. The hybrids positive for anti-pre-S were cloned by limited dilution and selected clones were then expanded and injected intraperitoneally into pristane primed Balb/c mice to produce ascitic fluid.

The antibodies directed to the pre-S region encoded epitopes in mice sera and in hybridomas were detected by direct ELISA. The polyvinyl plates were coated with synthetic pre-S1 peptide amino acids 12-49 of ay antigenic subtype or recombinant HBsAg pre-S2 particles described by Michel et al., P.N.A.S., 81, 7708-7712 (1984) at a concentration of 1 $\mu$g/ml in PBS. The sera in PBS was incubated for 1-2 hours at 37°C, washed with PBS containing 0.05% Tween 20 and incubated with HRPO-labeled anti-mouse antibody (H+L) (Dako).

Monoclonal antibody 5a-19 was selected on the basis of its reactivity with synthetic pre-S1 peptide (amino acid sequence 12-49) of ay synthetic peptide. This monoclonal was unreactive with HBsAg particles lacking pre-S1 encoded protein, unreactive with serum HBV of ad antigenic subtype and recognized pre-S1 amino acid sequence 35-53 in a strictly subtype specific manner. Furthermore, the reactivity of this monoclonal antibody to the large 39/43 kDA HBV protein was confirmed by Western Blot.

EXAMPLE 4

Preparation of Monoclonal Antibodies to the preS binding protein

HBV-binding protein prepared according to Example 2 was utilized to immunize Balb/c mice. 0.5 ml samples were emulsified in Freund's adjuvant and injected subcutaneously, followed by two intramuscular injections with incomplete Freund's adjuvant given at 10-days intervals. A final injection of the HBV-binding factor was administered 3 days before fusion with NSO myeloma cells. The monoclonal antibodies were prepared according to the procedure of Kohler and Milstein Nature, 256, 495 (1975) and the hybridomas were screened for the presence of antibodies against HBV-binding factor by ELISA on purified antigen coated plates using HRPO-conjugated anti-mouse immunoglobulins (H + L)(Dako). Monoclonal antibody 9H11B3, representative for the group of antibodies tested recognized the fractions with HBV-binding activity and was deposited at the C.N.C.M. under accession No. I1304 on May 7, 1993.

Ascites were produced by intraperitoneal injections of the positive hybridoma cells to mice which were pretreated with 0.5 ml of pristane.

DOT BLOT ANALYSIS

Fractions which were purified from HPLC were tested for their reactivity with anti-HBV-binding factor monoclonal antibody isolated as described above by dot-blot. Ten microliter aliquots of the samples were deposited on a nitrocellulose membrane and air-dried. The membranes were postcoated overnight with 5% skim milk, washed and incubated with anti-HBV-binding factor antibody diluted in skim milk. The antibody bound to the strips was reacted with HRPO-labeled anti-mouse Ig (H + L) (Dako) and revealed by enhanced chemiluminescent detection system (ACL Western Blotting Detection Kit, Amersham, Little Chalfont, UK).

IMMUNOHISTOCHEMISTRY

Liver tissue was obtained from normal human liver upon surgical resection for a benign tumor and liver samples of Peking duck, chicken and woodchuck were provided by L. Cova (INSERM U 271, Lyon, France). The liver tissues were embedded in OCT, quickly frozen in liquid nitrogen and stored at -80°C until use. Serial 5 μm thick sections were then cut, fixed for 5 minutes with cold acetone and air dried. Immunohistochemical staining was carried out according to the avidin-biotin peroxidase complex method using the Vectastain ABC elite kit (Vector Labs. Burlingame, CA) according to the instructions of the manufacturer. To block unspecific background, sections were preincubated in 3% normal sheep serum diluted in PBS. Sections were then incubated with the primary antibody 9H11B3 at a 1:200 dilution in PBS for 30 minutes at room temperature. Biotinylated mouse IgG (Vector) was used in a second step. Detection was carried out with 3-amino-9-ethyl carbazole as a chromogenic substrate. Controls included sections where the primary antibody 9H11B3 was omitted or replaced by irrelevant antibodies or PBS.

Immunohistochemical staining was also carried out on cultured hepatoma cells. HepG2 cells were grown under standard conditions in Dulbecco essential medium supplemented with 10% fetal calf serum. The cells were plated on 22 x 22 mm slides for 48 hours. After washing with PBS, the cells were fixed and stained according to the above described procedure.

While the invention has been described in terms of various preferred embodiments, the skilled artisan will appreciate that various modifications, substitutions omissions and changes may be made without departing from the scope thereof. Accordingly, it is intended that the scope of the present invention be limited solely by the scope of the following claims, including equivalents thereof.

**Claims**

1. An HBV-binding factor characterized in that said HBV-binding factor inhibits the binding of the Hepatitis B virus to the membranes of human hepatocytes.

2. The HBV-binding factor of Claim 1, characterized in that said HBV-binding factor is isolatable from human sera with or in the form of a glycoprotein which has a molecular weight of about 50 kDa, has an isoelectric point of 7.13 and is immunologically related to a membrane component of human hepatocytes, but not immunologically related to hepatocytes of species that are not susceptible to Hepatitis B virus infection.

3. The HBV-binding factor according to any one of Claims 1 or 2, wherein said HBV-binding factor interacts with the pre-S1 and pre-S2 epitopes of the viral envelope proteins located within the domains involved in the recognition of hepatocytes.

4. The HBV-binding factor according to any one of Claims 1 to 3, wherein said HBV-binding factor comprises N-acetylgalactosamine and N-acetylglucosamine residues, requires intact disulfide bonds, hydrophobic bonds and additional non-covalent linkages to maintain the necessary conformation for Hepatitis B virus recognition, represents a soluble form or fragment of a cellular Hepatitis B virus receptor(s), is stable at 37 C and is not a protease degrading pre-S determinants.

5. An HBV-binding factor which can be isolated from human sera with or in the form of a glycoprotein and inhibits the binding of the Hepatitis B virus to the membranes of human hepatocytes obtainable by:
(a) precipitating human sera with ammonium sulfate to produce globulins;
(b) dialyzing said precipitated globulins to remove the ammonium sulfate;
(c) placing said dialyzed globulins over an anion-exchange column particularly a DEAE-cellulose column;
(d) eluting fractions containing HBV-binding factor from said anion-exchange column;
(e) incubating said eluted fractions containing HBV-binding factor at 4°C for at least two weeks;
(f) placing said incubated fractions over a gel filtration column, particularly a Sephacryl S-200 column;
(g) eluting said fractions containing HBV-binding factor from said gel filtration column;
(h) adsorbing said eluted fractions from said gel filtration column onto beads preadsorbed with anti-Fc, followed by beads preadsorbed with anti-Fab; and
(i) isolating said HBV-binding factor from the beads.

6. An HBV-binding factor which interacts with the pre-S1 and pre-S2 epitopes involved in the binding of human HBV to hepatocyte membranes and as a consequence inhibits said binding which can be isolated from human sera by unmasking said HBV-binding factor activity by contacting said sera which has been previously precipitated with ammonium sulfate and then dialyzed with a monoclonal antibody produced by hybridoma number 9H11B3 deposited at the C.N.C.M. under accession No. I1304 on May 7, 1983 wherein said monoclonal antibody has been previously immobilized on an insoluble support, preferably an affinity resin to provide a complex of said HBV-binding factor with said monoclonal antibody, removing said non-fixed components and recovering said HBV-binding factor from said complex.

7. An HBV-binding factor, characterized in that said HBV-binding factor is capable of binding to monoclonal antibody 9H11B3 deposited at the C.N.C.M. on May 7, 1993 under accession No. I1304.

8. An HBV-binding factor characterized in that said HBV-binding factor is immunologically related to a membrane component of human hepatocytes, but not immunologically related to hepatocytes of species which are not susceptible to Hepatitis B virus, which prevents the binding of human HBV to hepatocyte membranes and which interacts with the pre-S1 and pre-S2 epitopes of the viral envelope proteins located within the domains involved in the recognition of hepatocytes.

9. Use of the HBV-binding factor according to any one of Claims 1 to 8 for the treatment and/or prevention of HBV-related liver diseases, in particular for the treatment of chronic active hepatitis, hepatocellular carcinoma or cirrhosis of the liver.

10. Use of the HBV-binding factor according to any one of Claims 1 to 7 as a reagent for the study of the interaction of Hepatitis B virus or parts thereof with the membrane.

Fig. 1

Fig. 2

Fig. 3A

Fig. 3B

Fig. 4

Fig. 5

Fig. 6

Fig. 7

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | VIROLOGY vol. 187, 1992, NEW YORK,USA pages 211 - 222 PETIT ET AL 'PRES1-SPECIFIC BINDING PROTEINS AS POTENTIAL RECEPTORS FOR HEPATITIS B VIRUS IN HUMAN HEPATOCYTES' | 1,9,10 | C07K15/04 C12P21/08 A61K37/02 |
| A | * page 211,summary * * page 216, paragraph 3 - page 217, paragraph 1 * * page 219, paragraph 3 - page 221, paragraph 6 * | | |
| | --- | | |
| D,X | JOURNAL OF VIROLOGY vol. 63, 1989, BALTIMORE,USA pages 1981 - 1988 PONTISSO ET AL 'HUMAN LIVER PLASMA MEMBRANES CONTAIN RECEPTORS FOR THE HEPATITIS B VIRUS PRE-S1 REGION AND,VIA POLYMERIZED HUMAN SERUM ALBUMIN,FOR THE PRE-S2 REGION' | 1,9,10 | |
| D,A | * page 1981,summary * * page 1987, paragraph 2 * | | TECHNICAL FIELDS SEARCHED (Int. Cl.5) |
| | --- | | |
| D,X | JOURNAL OF EXPERIMENTAL MEDICINE vol. 175, 1992, NEW YORK,USA pages 461 - 469 NEURATH ET AL 'SEARCH FOR HEPATITIS B VIRUS CELL RECEPTORS REVEALS BINDING SITES FOR INTERLEUKIN 6 ON THE VIRUS ENVELOPE PROTEIN' | 1,9,10 | C07K C12P A61K |
| D,A | * page 461,summary * page 464, paragraph 3 - page 465, paragraph 2 * | | |
| | --- | | |
| | -/-- | | |

The present search report has been drawn up for all claims

| Place of search | Date of completion of the search | Examiner |
|---|---|---|
| THE HAGUE | 28 SEPTEMBER 1993 | SITCH W.D.C. |

EPO FORM 1503 03.82 (P0401)

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,X | JOURNAL OF GENERAL VIROLOGY vol. 73, 1992, COLCHESTER,ENGLAND pages 2041 - 2045 PONTISSO ET AL 'THE PRES1 DOMAIN OF HEPATITIS B VIRUS AND IGA CROSS-REACT IN THEIR BINDING TO THE HEPATOCYTE SURFACE' | 1,9,10 | |
| D,A | * page 2041,summary * * page 2043, paragraph 2 - page 2044, paragraph 1 * * page 2044, paragraph 3 -paragraph 4 * | | |
| D,X | JOURNAL OF EXPERIMENTAL MEDICINE vol. 175, 1992, NEW YORK,USA pages 1195 - 1205 FRANCO ET AL 'TRANSFERRIN RECEPTOR MEDIATES UPTAKE AND PRESENTATION OF HEPATITIS B ENVELOPE ANTIGEN BY T LYMPHOCYTES' | 1,9,10 | |
| D,A | * page 1195,summary * page 1198, paragraph 2 * | | |

TECHNICAL FIELDS
SEARCHED (Int. Cl.5 )

The present search report has been drawn up for all claims

| Place of search | Date of completion of the search | Examiner |
|---|---|---|
| THE HAGUE | 28 SEPTEMBER 1993 | SITCH W.D.C. |

EPO FORM 1503 03.82 (P0401)